# EUROPEAN PATENT APPLICATION

(11) **EP 4 050 317 A1**
(43) Date of publication of application: **31.08.2022**
(21) Application number: 20894141.9
(22) Date of filing: 18.11.2020
(51) Int. Cl.: G01N 1/10, G01N 33/2028

(54) **METHOD FOR EXTRACTING PRECIPITATE AND/OR INCLUSION, METHOD FOR QUANTITATIVE ANALYSIS OF PRECIPITATE AND/OR INCLUSION, ELECTROLYTE, AND METHOD FOR PRODUCING REPLICA SAMPLE**

(30) Priority: 25.11.2019 JP 2019212278
(71) Applicant: JFE Steel Corporation, Tokyo 100-0011 (JP)
(72) Inventor: SUGAWARA, Seiya, Tokyo 100-0011 (JP); SAEKI, Michitoshi, Tokyo 100-0011 (JP); WATANABE, Manabu, Tokyo 100-0011 (JP)
(74) Representative: Haseltine Lake Kempner LLP
(86) International application number: PCT/JP2020/042956
(87) International publication number: WO 2021/106705

(57) **Abstract**

A precipitate and/or an inclusion in a metal material is extracted by electrolysis using an electrolyte solution. The electrolyte solution contains an adsorbent that is adsorbed to a surface of the precipitate and/or a surface of the inclusion. The extracted precipitate and/or the inclusion can be quantitatively analyzed with high accuracy.

## Description

### TECHNICAL FIELD

The present invention relates to a method of extracting a precipitate and/or an inclusion, a method of quantitative analysis of a precipitate and/or an inclusion, an electrolyte solution, and a method of preparing a replica sample.

### BACKGROUND ART

A precipitate and/or an inclusion (hereinafter, also referred to as "precipitate and the like") present in a metal material, depending on their abundance, has a significant influence on properties (for example, fatigue properties, hot workability, cold workability, deep drawability, machinability, electromagnetic properties, and the like) of the metal material.

In particular, when the metal material is a steel material, a technique for improving properties of the steel material using a trace amount of the precipitate and a technique for controlling the form of an inclusion have been remarkably developed in recent years.

Accordingly, the precipitate and the like are strictly controlled in a process of producing the steel material. For this purpose, it is necessary to quantitatively analyze the precipitate and the like with high accuracy.

In general, in order to quantitatively analyze the precipitate and the like in the metal material, first, the precipitate and the like are extracted. Thereafter, the extracted precipitate and the like are collected by filtration using a filter and subjected to quantitative analysis.

Methods of extracting the precipitate and the like can be roughly classified into an acid dissolution method, a halogen method, and an electrolytic dissolution method.

Among these methods, an electrolytic dissolution method (see Patent Literature 1) of extracting the precipitate and the like in the metal material by electrolysis is often used because the precipitate and the like can be stably extracted.

In addition, in recent years, as the metal material becomes more highly functional, controlling a metal structure or improving characteristics such as strength by using an extremely fine precipitate of 100 nm or less and the like has been performed on an industrial scale. In this case, it is required to quantitatively analyze the extremely fine precipitate and the like, and to observe and grasp the form thereof using, for example, a transmission electron microscope.

### CITATION LIST

### PATENT LITERATURE

Patent Literature 1: JP 2010-151695 A

### SUMMARY OF INVENTION

### TECHNICAL PROBLEMS

When the precipitate and the like in the metal material are extracted by electrolysis (electro extraction) and the extracted precipitate and the like are quantitatively analyzed, the resulting quantitative analysis value may greatly deviate from an expected value.

The present invention has been made in view of the above points, and an object of the present invention is to provide a method of extracting a precipitate and/or an inclusion (precipitate and the like), which method would enable accurate quantitative analysis of the extracted precipitate and the like.

Furthermore, another object of the present invention is to provide a method of quantitative analysis of a precipitate and the like using the extracting method, an electrolyte solution used in the extracting method, and a method of preparing a replica sample using the electrolyte solution.

### SOLUTION TO PROBLEMS

As a result of intensive studies by the present inventor, it was found that the above objects are achieved by adopting the following configuration.

That is, the present invention provides the following [1] to [15].
[1] A method of extracting a precipitate and/or an inclusion, comprising extracting the precipitate and/or the inclusion in a metal material by electrolysis using an electrolyte solution, wherein the electrolyte solution contains an adsorbent that is adsorbed to a surface of the precipitate and/or a surface of the inclusion.
[2] The method of extracting a precipitate and/or an inclusion according to claim 1, wherein the adsorbent is adsorbed to a surface of a matrix metal of the metal material.
[3] The method of extracting a precipitate and/or an inclusion according to [1] or [2], wherein the electrolyte solution contains an agent that forms a complex with a matrix metal of the metal material.
[4] The method of extracting a precipitate and/or an inclusion according to any one of [1] to [3], wherein the adsorbent is a compound having at least one group selected from the group consisting of a thiol group, a sulfide group, and a disulfide group.
[5] The method of extracting a precipitate and/or an inclusion according to any one of [1] to [4], wherein a content of the adsorbent in the electrolyte solution is 0.1 g/L or more with respect to a base electrolyte solution containing an electrolyte and a solvent.
[6] The method of extracting a precipitate and/or an inclusion according to any one of [1] to [5], wherein the metal material is a steel material.
[7] A method of quantitative analysis of a precipitate and/or an inclusion, comprising quantitatively analyzing the precipitate and/or the inclusion extracted by the extracting method according to any one of [1] to [6].
[8] An electrolyte solution for extracting a precipitate and/or an inclusion in a metal material by electrolysis, the electrolyte solution comprising an adsorbent that is adsorbed to a surface of the precipitate and/or a surface of the inclusion.
[9] The electrolyte solution according to [8], wherein the adsorbent is adsorbed to a surface of a matrix metal of the metal material.
[10] The electrolyte solution according to [8] or [9], further comprising an agent that forms a complex with a matrix metal of the metal material.
[11] The electrolyte solution according to any one of [8] to [10], wherein the adsorbent is a compound having at least one group selected from the group consisting of a thiol group, a sulfide group, and a disulfide group.
[12] The electrolyte solution according to any one of [8] to [11], wherein a content of the adsorbent is 0.1 g/L or more with respect to a base electrolyte solution containing an electrolyte and a solvent.
[13] The electrolyte solution according to any one of [8] to [12], wherein the metal material is a steel material.
[14] A method of preparing a replica sample comprising: subjecting a surface of a metal material to an electro etching using the electrolyte solution according to any one of [8] to [13]; and transferring a precipitate and/or an inclusion present on the surface of the metal material after the electro etching to a conductive thin film.
[15] The method of preparing a replica sample according to [14], wherein the conductive thin film is a carbon vapor deposition film.

### ADVANTAGEOUS EFFECTS OF INVENTION

According to the present invention, the extracted precipitate and the like can be quantitatively analyzed with high accuracy.

### BRIEF DESCRIPTION OF DRAWINGS

FIG. 1A is an SEM image of a precipitate and the like of Inventive Example 1.
FIG. 1B is an SEM-EDS mapping image (Mn mapping image) of the precipitate and the like of Inventive Example 1.
FIG. 1C is an SEM-EDS mapping image (Cu mapping image) of the precipitate and the like of Inventive Example 1.
FIG. 2A is an SEM image of a precipitate and the like of Comparative Example 1.
FIG. 2B is an SEM-EDS mapping image (Mn mapping image) of the precipitate and the like of Comparative Example 1.
FIG. 2C is an SEM-EDS mapping image (Cu mapping image) of the precipitate and the like of Comparative Example 1.
FIG. 3A is a TEM bright field image of a precipitate and the like in Inventive Example 8.
FIG. 3B is an EDS spectrum obtained from a precipitate 1 in FIG. 3A.
FIG. 3C is an EDS spectrum obtained from a precipitate 2 in FIG. 3A.
FIG. 3D is an EDS spectrum obtained from a precipitate 3 in FIG. 3A.
FIG. 4A is a TEM bright field image of a precipitate and the like in Comparative Example 3.
FIG. 4B is an EDS spectrum obtained from a precipitate 4 in FIG. 4A.
FIG. 4C is an EDS spectrum obtained from a precipitate 5 in FIG. 4A.
FIG. 4D is an EDS spectrum obtained from a precipitate 6 in FIG. 4A.

### DESCRIPTION OF EMBODIMENTS

### [Findings by the present inventor]

As described above, when a precipitate and the like in a metal material are extracted by electrolytic dissolution method (electro extraction) and the extracted precipitate and the like are quantitatively analyzed, the resulting quantitative analysis value may greatly deviate from the expected value.

Specifically, for example, when a steel material is electrolyzed to extract the precipitate and the like such as MnS, an error in the quantitative analysis value thereof may occur in some cases.

When a metal material such as a steel material containing Cu is electrolyzed, Cu is eluted together with the matrix metal in an electrolyte solution.

The present inventor expected that when Cu is present in the electrolyte solution, a substitution reaction with Mn in MnS occurs to convert MnS to CuS.

As a result of intensive studies, the present inventor has found a method of blocking this substitution reaction by performing electrolysis using an electrolyte solution containing a specific adsorbent.

Specifically, the present inventor first added 100 g/L of 6-dibutylamino-1,3,5-triazine-2,4-dithiol as an adsorbent to an AA electrolyte solution described later.

Next, a steel sheet which was a steel material having the chemical composition (the balance being Fe and inevitable impurities) shown in Table 1 below was prepared. It was confirmed using a scanning electron microscope (SEM) that all precipitates and the like of the steel sheet were MnS.

The prepared steel sheet was mirror-polished, immersed in the electrolyte solution for 1 second, pulled up therefrom, then washed with methanol, and dried. An X-ray photoelectron spectroscopy (XPS) spectrum was obtained for an arbitrary region in a dried steel sheet surface to determine concentrations of C, N and S.

Similarly, for the adsorbent (that is, 6-dibutylamino-1,3,5-triazine-2,4-dithiol), the XPS spectrum was obtained to determine the concentrations of C, N and S.

The concentrations (unit: atom%) of the respective components in the steel sheet surface and the adsorbent are shown in Table 2 below.

### [Table 1]

**Table 1**

| Chemical composition [mass%] | | | | | |
|---|---|---|---|---|---|
| C | Si | Mn | P | S | Cu |
| 0.396 | 0.106 | 1.607 | 0.031 | 0.021 | 0.112 |

### [Table 2]

**Table 2**

| | Concentration [atom%] | | |
|---|---|---|---|
| | C | N | s |
| Steel sheet surface | 69 | 20 | 11 |
| Adsorbent | 65 | 24 | 12 |

As shown in Table 2 above, concentration ratios of the respective components were approximately equal between the steel sheet surface and the adsorbent. This shows that 6-dibutylamino-1,3,5-triazine-2,4-dithiol as the adsorbent is adsorbed to the steel sheet surface (more specifically, a surface of the matrix metal of the steel sheet and a surface of the precipitate and the like attached thereto).

Next, the present inventor performed constant current electrolysis on a steel sheet having the chemical composition shown in Table 1 above using the electrolyte solution to extract precipitates and the like. The extracted precipitates and the like (MnS) were dissolved using an acid, and Cu was quantitatively analyzed by inductively coupled plasma atomic emission spectrometry (ICP-AES).

As a result, an amount of Cu was as very small as 0.0001 mass% (see Inventive Example 1 described later). On the other hand, when the adsorbent was not added to the electrolyte solution, an amount of Cu was as large as 0.0048 mass% (see Comparative Example 1 described later).

Therefore, it could be seen that the adsorbent (6-dibutylamino-1,3,5-triazine-2,4-dithiol) prevented Cu in the electrolyte solution from coming into contact with the precipitate and the like (MnS), and that above-described substitution reaction could be suppressed.

Although the reason why the substitution reaction can be suppressed is not definitely clear, the present inventor considers as follows.

When the metal material is electrolyzed in the electrolyte solution in which the adsorbent is present, an adsorption reaction and an electrolytic reaction alternately and continuously proceed.

The adsorption reaction is a reaction in which the adsorbent is adsorbed onto the surface of the matrix metal of the metal material.

The electrolytic reaction is a reaction in which the matrix metal of the metal material and a metal such as Cu taking the form of a solid solution in the matrix metal are ionized and eluted into the electrolyte solution.

As a result of the adsorption reaction and the electrolytic reaction proceeding alternately and continuously, the adsorbent is adsorbed to the surfaces of the precipitate and the like exposed on the surface of the matrix metal of the metal material. Then, when the matrix metal around the precipitate and the like is dissolved, the precipitate and the like adhere to the surface of the matrix metal of the metal material while being covered with the adsorbent.

Thus, even if Cu ions in the electrolyte solution come close to the precipitate and the like, a film of the adsorbent present on the surface of the precipitate and the like prevents the precipitate and the like from coming into contact with the Cu ions, and the substitution reaction is suppressed.

The present invention has been made based on the above findings.

Hereinafter, an embodiment of the present invention (hereinafter, also simply referred to as the "present invention") will be described.

### [Metal material and the like]

In the following description, the "metal material" is not particularly limited, and examples thereof include steel materials such as a hot-rolled steel sheet and a cold-rolled steel sheet.

The "matrix metal" of the metal material is an element contained most in the metal material, and is, for example, Fe when the metal material is a steel material.

The "precipitate and/or inclusion" (precipitate and the like) in the metal material is not particularly limited, and examples thereof include (Mn, Cu)S and MnS when the metal material is a steel material.

### [Electrolyte solution]

The electrolyte solution of the present invention is an electrolyte solution for extracting a precipitate and/or an inclusion in the metal material by electrolysis, the electrolyte solution containing an adsorbent that is adsorbed to the surface of the precipitate and/or the surface of the inclusion.

### <Electrolyte>

The electrolyte solution of the present invention substantially contains an electrolyte.

The electrolyte is not particularly limited, a conventionally known electrolyte can be used, and examples thereof include tetramethylammonium chloride, sodium chloride, and potassium bromide.

### <Solvent>

The electrolyte solution of the present invention substantially contains a solvent.

As the solvent, a solvent of a conventionally known electrolyte solution can be used, examples thereof include a nonaqueous solvent and an aqueous solvent (water), and the nonaqueous solvent is preferable because the adsorbent is easily dissolved.

Examples of the nonaqueous solvent suitably include nonaqueous solvents having a hydroxy group such as methanol, ethanol, propanol, and butanol.

### <Agent>

The electrolyte solution of the present invention preferably contains an agent that forms a complex with the matrix metal of the metal material (hereinafter, the agent is also simply referred to as the "agent"). With this constitution, re-adhesion and re-precipitation of the matrix metal dissolved in the electrolyte solution on the surface of the metal material are suppressed.

The agent is not particularly limited, and examples thereof suitably include acetylacetone, salicylic acid, methyl salicylate, maleic acid, citric acid, and sodium citrate.

The content of the agent is not particularly limited, and is preferably 1% or more, more preferably 5% or more, still more preferably 10% or more in terms of a mass ratio with respect to the solvent since it is necessary to form a complex with the matrix metal.

On the other hand, from the viewpoint of economic rationality, environmental load, and the like, the content of the agent is preferably 50% or less, more preferably 30% or less in terms of a mass ratio with respect to the solvent.

### <Base electrolyte solution>

The electrolyte solution of the present invention is preferably composed mainly of a base electrolyte solution.

The base electrolyte solution contains at least the above-described electrolyte and solvent, and may further contain the above-described agent.

The base electrolyte solution is not particularly limited, and examples thereof include nonaqueous solvent electrolyte solutions such as an AA (acetylacetone-tetramethylammonium chloride-methanol) electrolyte solution, an MS (methyl salicylate-salicylic acid-tetramethylammonium chloride-methanol) electrolyte solution, or an MA (maleic anhydride-tetramethylammonium chloride-methanol) electrolyte solution; and water-solvent-based electrolyte solutions such as citric acid electrolyte solution and hydrochloric acid electrolyte solution.

### <Adsorbent>

The adsorbent contained in the electrolyte solution of the present invention is not particularly limited as long as it is a substance that is adsorbed to a surface of a precipitate and the like. The adsorbent is preferably adsorbed to the surface of the matrix metal of the metal material. In the meantime, since the metal material usually has a positive charge, it is more preferable that the adsorbent has a negative surface potential and has an unpaired electron.

A suitable example of such an adsorbent is a compound having at least one group (hereinafter, also referred to as a "specific group" for convenience) selected from the group consisting of a thiol group, a sulfide group, and a disulfide group.

The compound having a specific group preferably has an amino group as a substituent.

The amino group is not particularly limited, and examples thereof include primary amino groups; secondary amino groups such as allylamino groups and butylamino groups; and tertiary amino groups such as a diisopropylamino group, a dibutylamino group, a diisobutylamino group, a di(2-ethylhexyl) amino group, a diallylamino group, and a (4-vinylbenzyl-n-propyl) amino group. Among them, the tertiary amino group is preferable.

Examples of the compound having a specific group and an amino group include aminothiols such as 2-aminoethanethiol and 2-aminopropanethiol.

The compound having a specific group preferably has a bulky structure in order to efficiently cover the surfaces of the precipitate and the like. Specific examples of the bulky structure suitably include a triazine ring.

Specific examples of the compound having a specific group, an amino group, and a triazine ring include 6-diallylamino-1,3,5-triazine-2,4-dithiol, 6-(4-vinylbenzyl-n-propyl)amino-1,3,5-triazine-2,4-dithiol, and 6-dibutylamino-1,3,5-triazine-2,4-dithiol.

Among these compounds, 6-dibutylamino-1,3,5-triazine-2,4-dithiol is more preferable because it is easily dissolved in a solvent (for example, methanol) and excellent in workability.

The content of the adsorbent in the electrolyte solution of the present invention is preferably 0.1 g/L or more with respect to the base electrolyte solution. Within this range, the adsorbent easily covers the surface of the metal material and easily prevents the contact with Cu.

Because this effect is more excellent, the content of the adsorbent is more preferably 0.2 g/L or more, still more preferably 2 g/L or more, even more preferably 10 g/L or more, particularly preferably 50 g/L or more, and most preferably 100 g/L or more with respect to the base electrolyte solution.

On the other hand, although the upper limit is not particularly limited, since the adsorbent is generally expensive, and it is difficult to dissolve the entire amount, the content of the adsorbent is preferably 500 g/L or less with respect to the base electrolyte solution.

### [Extracting method]

A method of extracting a precipitate and/or an inclusion (hereinafter, the extracting method is also referred to as the "extracting method of the present invention" for convenience) of the present invention is a method of extracting a precipitate and/or an inclusion in a metal material by electrolysis using an electrolyte solution, and in the method of extracting a precipitate and/or an inclusion, the electrolyte solution contains the adsorbent that is adsorbed to the surface of the precipitate and/or the surface of the inclusion.

The electrolyte solution of the present invention described above is an electrolyte solution used in the extracting method of the present invention.

### <Preparation of test sample>

In the extracting method of the present invention, it is preferable that the metal material is first cut into a test piece having an appropriate size, and subjected to polishing, cleaning, drying, and the like. Hereinafter, the test piece of the metal material having been subjected to polishing and the like is also referred to as a "test sample".

When a precipitate and the like are quantitatively analyzed, it is preferable to measure a mass of the test sample before electrolysis.

### <Electrolysis>

Next, using the electrolyte solution of the present invention, electrolysis (constant potential electrolysis or constant current electrolysis) is performed using the test sample as an anode.

A mass of the test sample electrolyzed is not particularly limited, and usually about 0.1 to 1 g of the test sample is electrolyzed. The mass of the test sample electrolyzed can be appropriately adjusted based on the amount of the electrolyte solution, the electrolysis conditions, the type of the test sample (metal material), the estimated value of the amount of precipitates and the like, and the like.

During electrolysis, it is preferable to stir the electrolyte solution using a magnetic stirrer or the like. As a result, the adsorbent is uniformly dispersed in the electrolyte solution and easily comes into contact with the test sample.

During electrolysis, the precipitate and the like contained in the test sample adheres as electrolytic residues to a surface of the test sample without being eluted in the electrolyte solution.

In the precipitate adsorbed to the surface of the test sample, the substitution reaction with Cu in the electrolyte solution is suppressed by the function of the adsorbent.

### <Separation of precipitate and the like>

After a predetermined amount of electrolysis, a remaining part of the test sample is gently taken out from the electrolyte solution and immediately immersed in a dispersion liquid so as not to allow electrolytic residues (precipitate and the like) to adhere to the remaining part of the test sample to fall into the electrolyte solution.

Since the remaining part of the test sample is immersed in the dispersion liquid, electrolytic residues (precipitate and the like) adhering to the remaining part of the test sample are separated from the remaining part of the test sample and dispersed in the dispersion liquid.

The dispersion liquid is not particularly limited, and a conventionally known dispersion liquid can be used, and examples of the dispersion liquid include methanol.

In order to rapidly separate the entire amount of precipitates and the like from the remaining part of the test sample, it is preferable to ultrasonically shake the dispersion liquid in which the remaining part of the test sample is immersed.

When the entire amount of precipitates and the like is separated from the remaining part of the test sample, the remaining part of the test sample exhibits metallic luster, and thus the metallic luster is used as a guide for an ultrasonic shaking time.

Thereafter, the remaining part of the test sample is taken out from the dispersion liquid. The remaining part of the test sample thus taken out is preferably sufficiently washed with methanol and the like and dried.

When the precipitate and the like are quantitatively analyzed, the mass of the remaining part of the test sample thus dried is measured and subtracted from the mass of the test sample before electrolysis to determine a mass of the test sample electrolyzed.

In one separation step (immersion and preferably ultrasonic shaking), the remaining part of the test sample may not exhibit metallic luster in some cases.

Specifically, for example, that is a case where the precipitate and the like cannot be separated in one separation step because of a large amount of precipitates and the like, and it is assumed that the precipitate and the like remain on a surface of the remaining part of the test sample.

In this case, it is preferable to separately provide a dispersion liquid and repeat the separation step a plurality of times until the remaining part of the test sample exhibits metallic luster.

### <Collection of precipitate and the like>

The dispersion liquid from which the remaining part of the test sample is taken out (dispersion liquid in which the precipitate and the like are dispersed) is filtered (for example, vacuum filtration) using a filter to collect the precipitate and the like on the filter.

Among the precipitate and the like, those not larger than several tens of nanometers are likely to aggregate. Thus, the pore size of the filter used for collecting the precipitate and the like does not need to be equal to or smaller than an assumed size of the precipitate and the like, and may be selected according to the average particle size of the precipitate and the like.

### [Quantitative analysis method]

A method of quantitative analysis of a precipitate and/or an inclusion of the present invention (hereinafter, this method is also referred to as the "quantitative analysis method of the present invention" for convenience) is a method of quantitative analysis of a precipitate and/or an inclusion, in which the precipitate and/or the inclusion extracted by the extracting method of the present invention described above are quantitatively analyzed.

In the quantitative analysis method of the present invention, it is preferable that the precipitate and the like extracted by the extracting method of the present invention described above are dissolved according to a standard method and quantitatively analyzed.

For the dissolution of the precipitate and the like, a conventionally known acid aqueous solution or alkali aqueous solution can be used, and is appropriately selected according to a target element to be quantitatively analyzed.

Examples of the quantitative analysis method suitably include inductively coupled plasma atomic emission spectrometry (ICP-AES), inductively coupled plasma mass spectrometry (ICP-MS), and atomic absorption spectrometry.

The electrolyte solution of the present invention can be used for preparing a replica sample for observing the precipitate and the like with an electron microscope. Accordingly, the precipitate and the like can be analyzed with high accuracy.

### [Method of preparing replica sample]

A method of preparing a replica sample of the present invention is a method of preparing a replica sample in which a surface of a metal material is subjected to an electro etching using the electrolyte solution of the present invention described above, and the precipitate and/or the inclusion present on the surface of the metal material after the electro etching are transferred to a conductive thin film.

### <Preparation of test sample>

First, it is preferable to polish the surface of the metal material. The polishing method is not particularly limited, and a polishing method may be selected according to the material, characteristics, and the like of the metal material according to a conventional method.

### <Electro etching>

Next, the surface of the test sample (metal material) is subjected to an electro etching using the electrolyte solution of the present invention. As a result, a precipitate and the like are exposed on the surface of the test sample.

During electro etching, it is preferable to stir the electrolyte solution using a magnetic stirrer or the like. With this constitution, the adsorbent is uniformly dispersed in the electrolyte solution and easily comes into contact with the test sample.

An electro etching amount can be appropriately adjusted based on the amount of the electrolyte solution, the electrolysis conditions, the type of the test sample (metal material), the estimated value of the amount of precipitates and the like, and the like.

### <Transfer>

Next, the precipitate and the like exposed on the surface of the metal material by electro etching are transferred to a conductive thin film and collected. The transfer collection method may follow a conventional method, and examples thereof include a two-stage replica method generally used for metal materials.

The two-stage replica method is outlined as follows.

First, an organic film formed from acetyl cellulose and the like is softened and dissolved using methyl acetate or the like and then fused to the surface of the metal material after electro etching. Thereafter, the organic film is peeled off from the metal material. As a result, the precipitate and the like are transferred and collected on a surface of the organic film.

Next, a conductive thin film is formed on the surface of the organic film having been peeled on which surface the precipitate and the like are transferred and collected. Thereafter, the organic film is dissolved using an organic solvent such as methyl acetate. As a result, a sample (replica sample) in which the precipitate and the like are transferred and collected on the conductive thin film is obtained.

The conductive thin film is preferably a carbon vapor deposition film for the following reasons.

In general, characteristic X-rays are used for composition analysis of the collected precipitate and the like. Since carbon has a small absorption coefficient of X-rays, it is easy to acquire the characteristic X-rays from the precipitate and the like by using the carbon vapor deposition film as the conductive thin film.

In addition, in the case of observing and analyzing extremely fine precipitates and the like, a transmitted electron beam is used; since carbon easily transmits the electron beam, by using the carbon vapor deposition film as the conductive thin film, it is easy to determine the precipitates and the like.

When the conductive thin film is the carbon vapor deposition film, a film thickness of the carbon vapor deposition film is not particularly limited, and is, for example, 5 nm or more and 30 nm or less.

The film thickness of the carbon vapor deposition film is preferably more than 10 nm and less than 20 nm. With this constitution, the replica sample has a strength capable of holding the precipitate and the like (and unavoidably extracted adsorbent) transferred from the surface of the metal material while having sufficient flexibility. The film thickness of the carbon vapor deposition film is more preferably 11 nm or more, still more preferably 12 nm or more.

The film thickness of the carbon vapor deposition film is measured by interference spectroscopy.

The prepared replica sample is subjected to, for example, observation using an electron microscope.

At this time, the conductive thin film of the replica sample is held by, for example, a mesh. The mesh is not particularly limited, and the material of the mesh is preferably different from an element contained in the precipitate and the like.

### EXAMPLES

Hereinafter, the present invention will be specifically described with reference to Examples. However, the present invention is not limited to Examples described below.

### [Test 1]

### <Inventive Example 1>

In Inventive Example 1, a precipitate and the like in a test sample were extracted and quantitatively analyzed using an electrolyte solution A described later. Details thereof are described below.

### <<Preparation of test sample>>

A steel ingot having a chemical composition (the balance being Fe and inevitable impurities) shown in Table 1 above was produced by vacuum melting. The produced steel ingot was heated to 1200°C and then hot-rolled to produce a hot-rolled steel sheet having a thickness of 3 mm.

A sample for cross-section observation was collected from the produced hot-rolled steel sheet. When the collected sample was observed using a scanning electron microscope (SEM), it was confirmed that all precipitates and the like were MnS.

Next, a test piece having a size of 30 mm × 30 mm was collected from the produced hot-rolled steel sheet, and the surface was polished to obtain a test sample.

### <<Electro extraction>>

The electrolyte solution A (content of adsorbent with respect to base electrolyte solution: 100 g/L) was prepared by adding 10 g of 6-dibutylamino-1,3,5-triazine-2,4-dithiol as an adsorbent to 100 mL of a 10% AA electrolyte solution (10 mass% acetylacetone-1 mass% tetramethylammonium chloride-methanol).

Using the prepared electrolyte solution A, a test sample was subjected to constant current electrolysis under the condition of a current density of 20 mA/cm².

After 0.1 g of the test sample was electrolyzed, the remaining part of the test sample taken out from the electrolyte solution A was immersed in methanol as a dispersion liquid, ultrasonic shaking was applied to it for 2 minutes, and it was confirmed that metallic luster appeared in the remaining part of the test sample. In this manner, the precipitate and the like adhering to the remaining part of the test sample were separated and dispersed in the dispersion liquid. Thereafter, the remaining part of the test sample was taken out from the dispersion liquid.

Next, the dispersion liquid from which the remaining part of the test sample was taken out was filtered through a filter having a pore size of 0.2 µm, and the precipitate and the like were collected on the filter. As the filter, a track etched membrane filter having a smooth surface was used so as to facilitate SEM observation (described later).

### <<Quantitative analysis>>

The collected precipitate and the like were put in a beaker together with the filter, 20 mL of nitric acid was added thereto, and they were heated at 100°C for 30 minutes to dissolve the precipitate and the like. After the heating, the filter was taken out from the beaker, and nitric acid adhering to the filter was washed away with pure water.

The liquid in the beaker was quantitatively analyzed by ICP-AES using an ICP emission spectrometer (ICPS-8100, manufactured by Shimadzu Corporation) to determine a Mn amount and a Cu amount (unit: mass%) of the precipitate and the like collected on the filter. The obtained value is a value converted into a concentration in steel. The result is shown in Table 3 below.

### <<SEM observation>>

A filter (different from the filter used for the quantitative analysis described above) on which the precipitate and the like were collected was dried with a dryer, and then carbon vapor deposition was performed to impart conductivity to the filter.

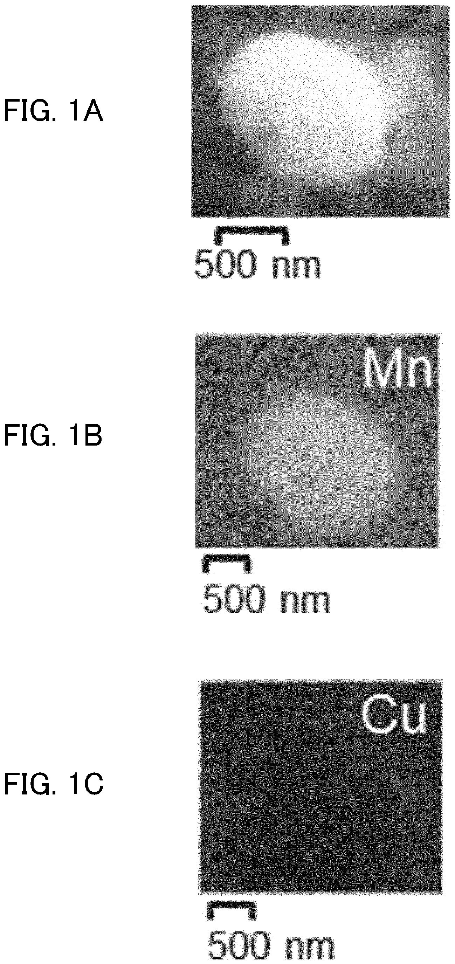

Thereafter, the precipitate and the like on the filter were observed using an SEM. At that time, an SEM-EDS mapping image of the precipitate and the like was acquired using an energy dispersive X-ray spectroscopy (EDS) apparatus attached to the SEM. The results are shown in FIGS. 1A to 1C.

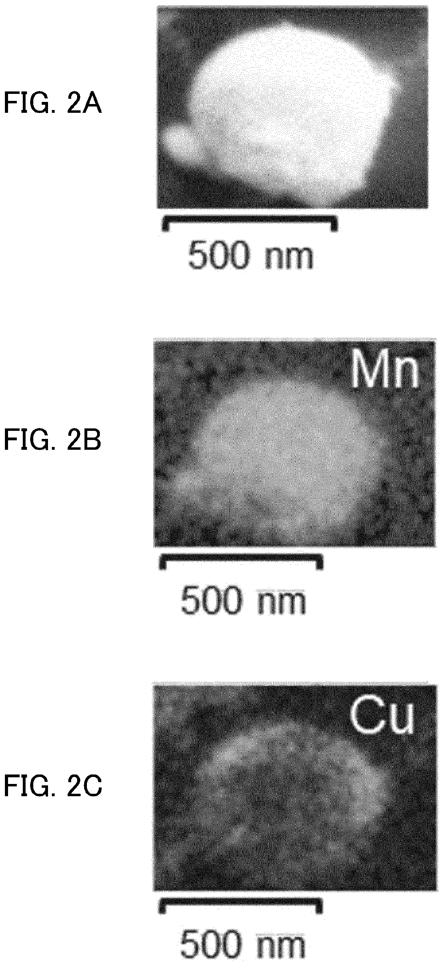

FIG. 1A shows an SEM image of precipitate and the like. FIG. 1B shows a Mn mapping image. FIG. 1C shows a Cu mapping image (the same applies to FIGS. 2A to 2C described later).

### <Comparative Example 1>

Use was made of 100 mL of a 10% AA electrolyte solution as an electrolyte solution B without adding an adsorbent.

The electro extraction, the quantitative analysis, and the SEM observation were performed in the same manner as in Inventive Example 1 except that the electrolyte solution B was used instead of the electrolyte solution A. The result of the quantitative analysis is shown in Table 3 below. The results of the SEM observation are shown in FIGS. 2A to 2C.

### [Table 3]

**Table 3**

| | Electrolyte solution | Mn amount [mass%] | Cu amount [mass%] |
|---|---|---|---|
| Inventive Example 1 | A | 0.0101 | 0.0001 |
| Comparative Example 1 | B | 0.0037 | 0.0048 |

### <Summary of evaluation results>

First, regarding Comparative Example 1, FIGS. 2A to 2C (SEM-EDS mapping images of the precipitate and the like of Comparative Example 1) show that a large amount of Cu was present on the surface of the precipitate and the like. This fact reveales that the substitution reaction of MnS by Cu occurs.

Referring to Table 3 above, in Comparative Example 1, the Cu amount in the precipitate and the like was 0.0048 mass%, which was a value higher than the Mn amount (0.0037 mass%).

On the other hand, in Inventive Example 1, FIGS. 1A to 1C (SEM-EDS mapping images of the precipitate and the like of Inventive Example 1) show that the precipitate and the like were only MnS, and Cu could not be confirmed.

As shown in Table 3 above, in the precipitate and the like in Inventive Example 1, the Mn amount was 0.0101 mass%, the Cu amount was 0.0001 mass%, and Cu was hardly detected.

Therefore, Inventive Example 1 can be evaluated as having the collected precipitates and the like quantitatively analyzed with higher accuracy than in Comparative Example 1.

### [Test 2]

Next, the amount of the adsorbent contained in the electrolyte solution was changed, and the effect of the present invention in that case was confirmed.

### <Inventive Examples 2 to 5>

Electrolyte solutions C to F were prepared by adding 0.02 g, 0.2 g, 1 g, or 5 g of an adsorbent (6-dibutylamino-1,3,5-triazine-2,4-dithiol) to 100 mL of an MA electrolyte solution (10 mass% maleic anhydride-1 mass% tetramethylammonium chloride-methanol).

Using the electrolyte solutions C to F, the electro extraction and the quantitative analysis were performed in the same manner as in Inventive Example 1. The result of the quantitative analysis is shown in Table 4 below.

### [Table 4]

**Table 4**

| | Electrolyte solution | Adsorbent Content [g] | Adsorbent Content [g/L] | Mn amount [mass%] |
|---|---|---|---|---|
| Inventive Example 2 | C | 0.02 | 0.2 | 0.0082 |
| Inventive Example 3 | D | 0.2 | 2 | 0.0102 |
| Inventive Example 4 | E | 1 | 10 | 0.0103 |
| Inventive Example 5 | F | 5 | 50 | 0.0101 |

### <Summary of evaluation results>

As shown in Table 4 above, when any electrolyte solution was used, the Mn amount in the precipitate and the like showed a value higher than that in Comparative Example 1 and equal to that in Inventive Example 1.

Therefore, it can be determined that even when the content of the adsorbent in the electrolyte solution was extremely low, the collected precipitates and the like could be quantitatively analyzed with high accuracy.

### [Test 3]

Next, the type of the adsorbent contained in the electrolyte solution was changed, and the effect of the present invention in that case was confirmed.

### <Inventive Example 6>

An electrolyte solution G (content of adsorbent with respect to base electrolyte solution: 50 g/L) was prepared by adding 5 g of 2-aminoethanethiol as an adsorbent to 100 mL of the 10% AA electrolyte solution.

Using the electrolyte solution G, the electro extraction and the quantitative analysis were performed in the same manner as in Inventive Example 1. The result of the quantitative analysis is shown in Table 5 below.

### [Table 5]

**Table 5**

| | Electrolyte solution | Mn amount [mass%] | Cu amount [mass%] |
|---|---|---|---|
| Inventive Example 6 | G | 0.0081 | 0.0020 |

### <Summary of evaluation results>

As shown in Table 5 above, in Inventive Example 6 in which the electrolyte solution G was used, the Cu amount was reduced as compared with Comparative Example 1, and the Mn amount was equivalent to that in Inventive Example 1.

Therefore, it can be determined that also in Inventive Example 6 using the adsorbent different from Inventive Example 1, the collected precipitate and the like could be quantitatively analyzed with high accuracy.

### [Test 4]

In recent years, the use of scrap has been promoted in the respective industries including the steel industry.

Scrap contains precious metals that cannot be completely removed, and these precious metals (in particular, silver) are frequently contained in a metal material such as a steel material.

Since silver (Ag) is a metal element that easily reacts, it is expected that an error in the quantitative analysis value of the extracted precipitate and the like increases due to reaction with the precipitate and the like exposed on the surface of the metal material in electrolysis.

However, by performing the electro extraction using the electrolyte solution of the present invention, the adsorbent is adsorbed to the surface of the precipitate and the like, and the reaction with silver can be suppressed. Hereinafter, this effect was confirmed.

### <Inventive Example 7>

An electrolyte solution H (content of adsorbent with respect to base electrolyte solution: 100 g/L) was prepare by adding 10 g of an adsorbent (6-dibutylamino-1,3,5-triazine-2,4-dithiol) and further 1 mL of a silver standard solution (concentration of silver: 1 mg/L) produced by KANTO KAGAKU to 100 mL of an MA electrolyte solution.

### <Comparative Example 2>

An electrolyte solution I was prepared by adding 1 mL of a silver standard solution (concentration of silver: 1 mg/L) produced by KANTO KAGAKU to 100 mL of an MA electrolyte solution.

Using the electrolyte solutions H and I, the electro extraction and the quantitative analysis were performed in the same manner as in Inventive Example 1. The result of the quantitative analysis is shown in Table 6 below.

### [Table 6]

**Table 6**

| | Electrolyte solution | Mn amount [mass%] | Cu amount [mass%] | Ag amount [mass%] |
|---|---|---|---|---|
| Inventive Example 7 | H | 0.0089 | 0.0009 | 0.0026 |
| Comparative Example 2 | I | 0.0041 | 0.0035 | 0.0500 |

### <Summary of evaluation results>

As shown in Table 6 above, an amount of Ag in Comparative Example 2 was 0.0500 mass%, and a large amount of silver not contained in the precipitate and the like was found in the analysis.

On the other hand, in Inventive Example 7, the Ag amount was as small as 0.0026 mass%, and the Mn amount and the Cu amount had values equivalent to those in Inventive Example 1.

Therefore, it can be determined that in Inventive Example 7, the collected precipitates and the like could be quantitatively analyzed with higher accuracy as compared with Comparative Example 2.

### [Test 5]

### <Inventive Example 8>

Using the same electrolyte solution A as in Inventive Example 1, a replica sample of the precipitate and the like present on the surface of the test sample was prepared. Details thereof are described below.

### <<Preparation of test sample>>

Molten steel having the chemical composition (the balance being Fe and inevitable impurities) shown in Table 7 below was subjected to vacuum degassing treatment, and then subjected to continuous casting to obtain a slab. Subsequently, the obtained slab was heated to 1150°C, scale was removed, and then the slab was roughly rolled to a sheet thickness of 40 mm. A surface layer of the roughly-rolled sheet obtained was cooled by a scale removal device, and the sheet was then finish-rolled to a thickness of 3.5 mm, and wound into a coil at 700°C to obtain a hot-rolled steel sheet. The surface of the obtained hot-rolled steel sheet after mirror polishing was observed using an SEM, and it was confirmed that MnS and TiMnS were contained.

### [Table 7]

**Table 7**

| Chemical composition [mass%] | | | | | | |
|---|---|---|---|---|---|---|
| C | Si | Mn | P | S | Ti | Cu |
| 0.002 | 0.02 | 0.15 | 0.013 | 0.011 | 0.035 | 0.05 |

### <<Preparation of replica sample>>

A 10 mm square steel piece was collected from the obtained hot-rolled steel sheet, only scale was removed using an acid, and a piece surface was mirror-polished. Thereafter, electro etching was performed on the steel piece using the electrolyte solution A (see Inventive Example 1) under conditions that an electrolytic thickness was 1 µm from the surface layer per one surface and the current density was 5 mA/cm².

An organic film (acetylcellulose) softened and dissolved using methyl acetate was fused to the surface of the steel piece after the electro etching. Thereafter, the organic film was peeled off to transfer and collect the precipitate and the like on the surface of the steel piece on the surface of the organic film.

Next, a carbon vapor deposition film having a film thickness of 15 nm was formed on the surface of the organic film after peeling on which surface the precipitate and the like were transferred and collected.

Thereafter, the organic film was dissolved using an organic solvent (methyl acetate). Subsequently, the carbon vapor deposition film was held using a commercially available Ni mesh (mesh size: 150 µm).

Thus, a sample (replica sample) in which the precipitate and the like on the surface of the steel piece were transferred and collected on the carbon vapor deposition film was prepared.

### <<Qualitative analysis>>

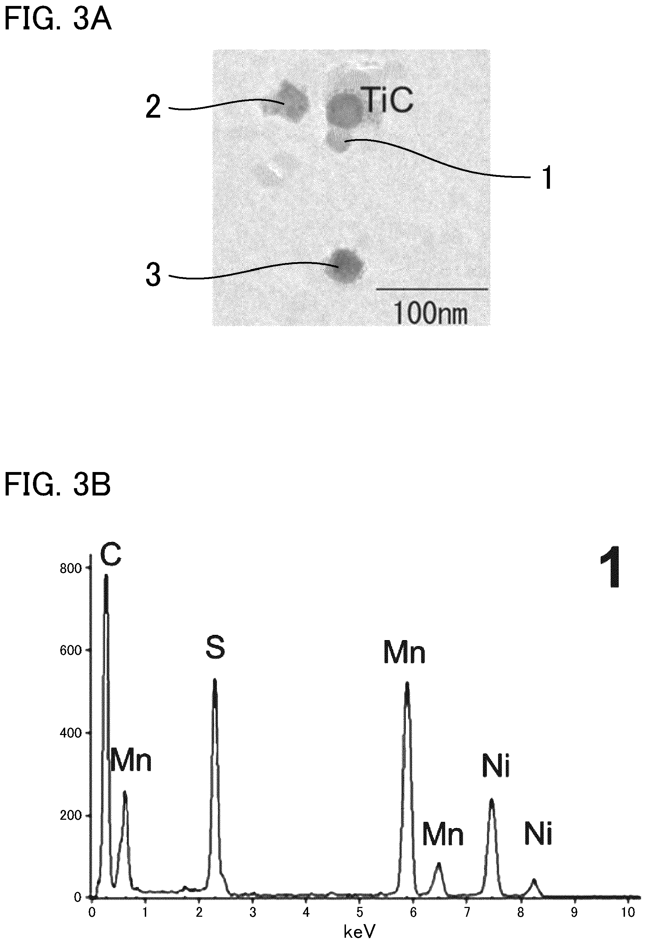

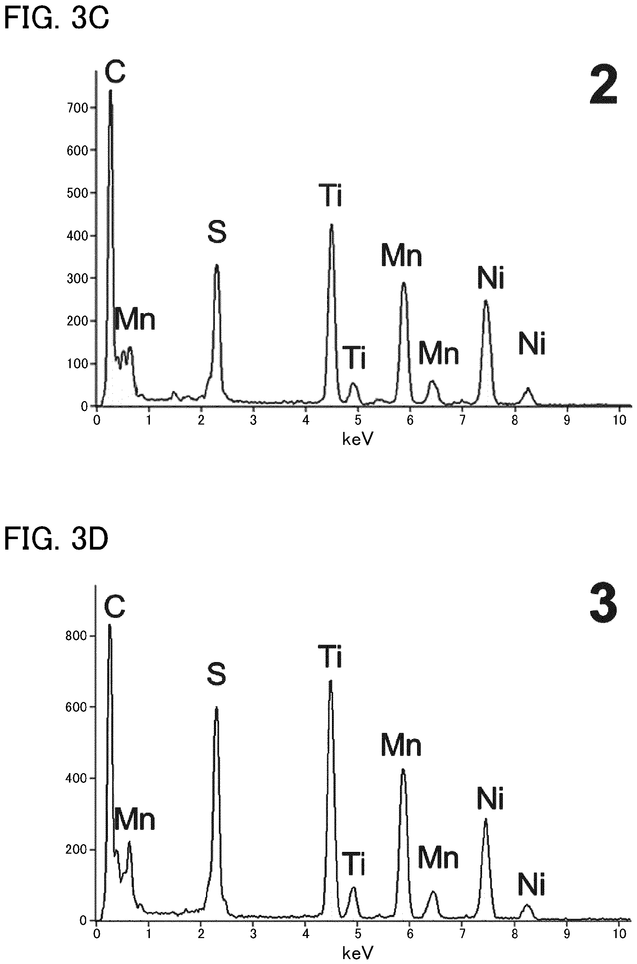

A surface of the prepared replica sample was observed using a transmission electron microscope (TEM) to obtain a TEM bright field image. The observation conditions were an acceleration voltage of 200 kV and an observation magnification of 10,000 times. In addition, the precipitate and the like were qualitatively analyzed using an EDS apparatus attached to a TEM. The results are shown in FIGS. 3A to 3D.

FIG. 3A is a TEM bright field image of the precipitate and the like in Inventive Example 8. FIGS. 3B to 3D are EDS spectra obtained from precipitates 1 to 3 in FIG. 3A, respectively.

### <Comparative Example 3>

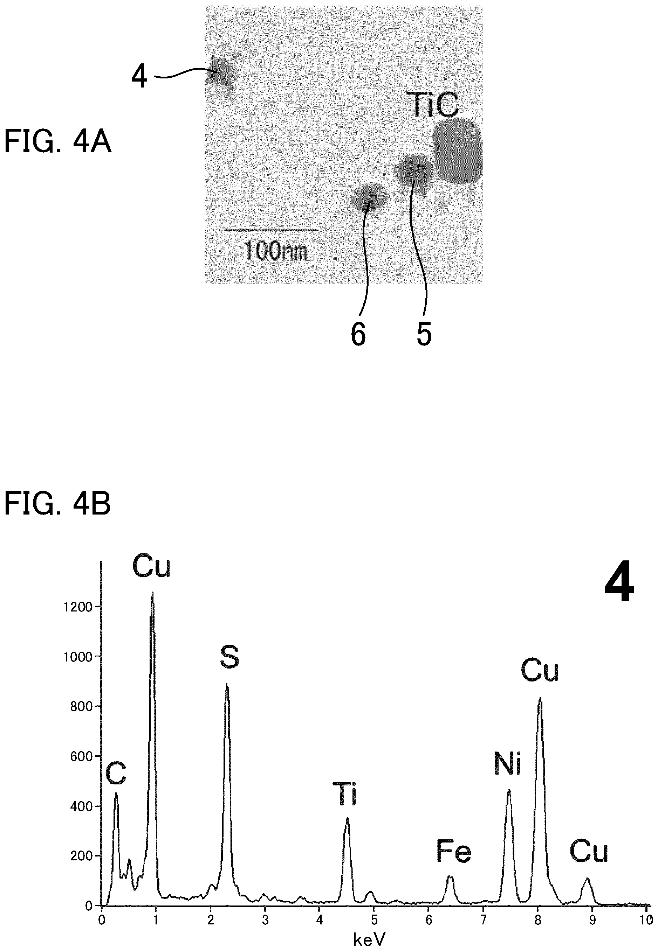

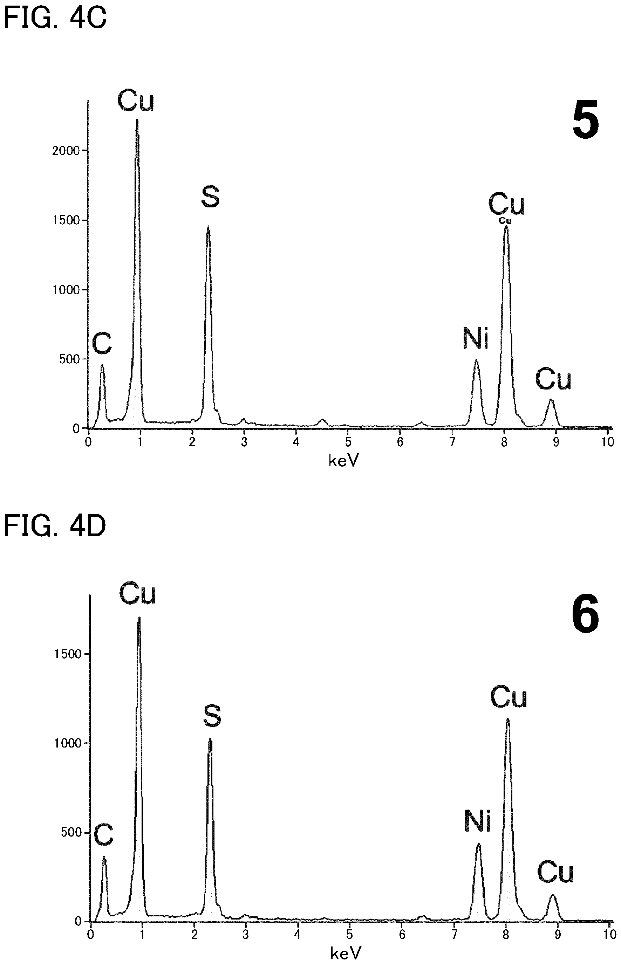

Preparation and qualitative analysis of the replica sample were performed in the same manner as in Inventive Example 8, except that electro etching was performed using the electrolyte solution B (see Comparative Example 1) instead of the electrolyte solution A. The results are shown in FIGS. 4A to 4D.

FIG. 4A is a TEM bright field image of the precipitate and the like in Comparative Example 3. FIGS. 4B to 4D are EDS spectra obtained from precipitates 4 to 6 in FIG. 4A, respectively.

### <Summary of evaluation results>

In Inventive Example 8, the precipitate and the like were MnS and TiMnS, and Cu could not be confirmed from the EDS spectra shown in FIGS. 3B to 3D.

On the other hand, in Comparative Example 3, it is found that the precipitate and the like are CuS and TiCuS from the EDS spectra shown in FIGS. 4B to 4D.

From this, it can be seen that when the adsorbent is not added to the electrolyte solution used for electro etching, the substitution reaction with Mn by Cu occurs.

Therefore, it can be determined that in Inventive Example 8, fine precipitates and the like extracted in the replica sample can be quantitatively analyzed with higher accuracy as compared with Comparative Example 3.

### [Test 6]

### <Inventive Examples 9 to 10>

Replica samples were prepared in the same manner as in Inventive Example 8 except that the film thickness of the carbon vapor deposition film was changed from 15 nm to 10 nm and 20 nm, respectively.

With respect to Inventive Example 8 and Inventive Examples 9 to 10 described above, the state of the carbon vapor deposition film was observed during the preparation of the replica sample.

Specifically, the state of the carbon vapor deposition film was observed at the time when the organic film (acetylcellulose) was dissolved using the organic solvent and the time when the carbon vapor deposition film was held using the Ni mesh.

Table 8 below shows "A" when an initial shape of the carbon vapor deposition film was maintained such that the carbon vapor deposition film could be subjected to observation with an electron microscope, and "B" when partial collapse of the carbon vapor deposition film was observed.

### [Table 8]

**Table 8**

| | Film thickness of carbon vapor deposition film [nm] | At the time when organic film was dissolved | At the time when held by mesh |
|---|---|---|---|
| Inventive Example 8 | 15 | A | A |
| Inventive Example 9 | 10 | B | - |
| Inventive Example 10 | 20 | A | B |

### <Summary of evaluation results>

As shown in Table 8 above, in Inventive Example 9 (film thickness of carbon vapor deposition film: 10 nm), partial collapse of the carbon vapor deposition film was observed at the time when the organic film was dissolved.

Next, in Inventive Example 10 (film thickness of carbon vapor deposition film: 20 nm), although collapse of the carbon vapor deposition film was not observed at the time when the organic film was dissolved, partial collapse of the carbon vapor deposition film was observed at the time when the carbon vapor deposition film was held by the Ni mesh.

On the other hand, in Inventive Example 8 (film thickness of carbon vapor deposition film: 15 nm), collapse of the carbon vapor deposition film was not observed at the both time points. Thus, the sample of Inventive Example 8 was found to be suitable as an observation sample for TEM, for example.

## Claims

1. A method of extracting a precipitate and/or an inclusion, comprising extracting the precipitate and/or the inclusion in a metal material by electrolysis using an electrolyte solution,
wherein the electrolyte solution contains an adsorbent that is adsorbed to a surface of the precipitate and/or a surface of the inclusion.

2. The method of extracting a precipitate and/or an inclusion according to claim 1, wherein the adsorbent is adsorbed to a surface of a matrix metal of the metal material.

3. The method of extracting a precipitate and/or an inclusion according to claim 1 or 2, wherein the electrolyte solution contains an agent that forms a complex with a matrix metal of the metal material.

4. The method of extracting a precipitate and/or an inclusion according to any one of claims 1 to 3, wherein the adsorbent is a compound having at least one group selected from the group consisting of a thiol group, a sulfide group, and a disulfide group.

5. The method of extracting a precipitate and/or an inclusion according to any one of claims 1 to 4, wherein a content of the adsorbent in the electrolyte solution is 0.1 g/L or more with respect to a base electrolyte solution containing an electrolyte and a solvent.

6. The method of extracting a precipitate and/or an inclusion according to any one of claims 1 to 5, wherein the metal material is a steel material.

7. A method of quantitative analysis of a precipitate and/or an inclusion, comprising quantitatively analyzing the precipitate and/or the inclusion extracted by the extracting method according to any one of claims 1 to 6.

8. An electrolyte solution for extracting a precipitate and/or an inclusion in a metal material by electrolysis,
the electrolyte solution comprising an adsorbent that is adsorbed to a surface of the precipitate and/or a surface of the inclusion.

9. The electrolyte solution according to claim 8, wherein the adsorbent is adsorbed to a surface of a matrix metal of the metal material.

10. The electrolyte solution according to claim 8 or 9, further comprising an agent that forms a complex with a matrix metal of the metal material.

11. The electrolyte solution according to any one of claims 8 to 10, wherein the adsorbent is a compound having at least one group selected from the group consisting of a thiol group, a sulfide group, and a disulfide group.

12. The electrolyte solution according to any one of claims 8 to 11, wherein a content of the adsorbent is 0.1 g/L or more with respect to a base electrolyte solution containing an electrolyte and a solvent.

13. The electrolyte solution according to any one of claims 8 to 12, wherein the metal material is a steel material.

14. A method of preparing a replica sample comprising:
subjecting a surface of a metal material to an electro etching using the electrolyte solution according to any one of claims 8 to 13; and transferring a precipitate and/or an inclusion present on the surface of the metal material after the electro etching to a conductive thin film.

15. The method of preparing a replica sample according to claim 14, wherein the conductive thin film is a carbon vapor deposition film.
